# EUROPEAN PATENT SPECIFICATION

(11) **EP 4 180 537 B1**
(45) Date of publication and mention of the grant of the patent: **10.06.2026**
(21) Application number: 21841657.6
(22) Date of filing: 13.07.2021
(51) Int. Cl.: G01N 33/569, C12Q 1/70

(54) **SIMPLE METHOD FOR SAMPLING MUCUS ADHERED TO NASAL MUCOSA SURFACE AND TEST KIT FOR DETECTING ANALYTE IN SAMPLE**
EINFACHES VERFAHREN ZUR PROBENAHME VON AN DER NASENSCHLEIMHAUTOBERFLÄCHE HAFTENDER SCHLEIM UND TESTKIT ZUM NACHWEIS EINES ANALYTS IN EINER PROBE
PROCÉDÉ SIMPLE D'ÉCHANTILLONNAGE DE MUCUS ADHÉRANT À UNE SURFACE DE MUQUEUSE NASALE ET KIT DE TEST POUR DÉTECTER UN ANALYTE DANS UN ÉCHANTILLON

(30) Priority: 16.07.2020 JP 2020122106
(43) Date of publication of application: 17.05.2023
(73) Proprietor: Denka Company Limited, Tokyo 103-8338 (JP)
(72) Inventor: MIYAZAWA Takashi, Gosen-shi, Niigata 959-1695 (JP); TIEN Chihfang, Gosen-shi, Niigata 959-1695 (JP)
(74) Representative: Wohlfahrt, Jan Günther
(86) International application number: PCT/JP2021/026239
(87) International publication number: WO 2022/014563

(56) References cited:
- JP-A- 2006 118 936
- JP-A- 2008 539 733
- JP-A- 2019 504 319
- JP-A- H1 031 021
- US-A1- 2005 048 465
- US-A1- 2007 184 495
- US-A1- 2009 275 015
- US-A1- 2015 079 039
- US-A1- 2015 224 246
- US-A1- 2018 110 499
- NOAH TERRY L. ET AL: "Chemokines in Nasal Secretions of Normal Adults Experimentally Infected with Respiratory Syncytial Virus", CLINICAL IMMUNOLOGY, vol. 97, no. 1, 1 October 2000 (2000-10-01), AMSTERDAM, NL, pages 43 - 49, XP093082313, ISSN: 1521-6616, DOI: 10.1006/clim.2000.4914
- VEL�ZQUEZ-G�MEZ MIGUEL ET AL: "Nasal lavages as a tool for monitoring exposure to organic pollutants", ENVIRONMENTAL RESEARCH, ACADEMIC PRESS, SAN DIEGO, CA, US, vol. 178, 6 September 2019 (2019-09-06), XP085832339, ISSN: 0013-9351, [retrieved on 20190906], DOI: 10.1016/J.ENVRES.2019.108726
- FUJIMOTO, C. ; KIDO, H. ; SAWABUCHI, T. ; MIZUNO, D. ; HAYAMA, M. ; YANAGAWA, H. ; TAKEDA, N.: "Evaluation of nasal IgA secretion in normal subjects by nasal spray and aspiration", AURIS NASUS LARYNX, TOKYO, AMSTERDAM, NL, vol. 36, no. 3, 1 June 2009 (2009-06-01), AMSTERDAM, NL , pages 300 - 304, XP026966647, ISSN: 0385-8146

## Description

### Technical Field

The present invention provides a method for collecting a sample of a mucosal fluid adhered to the mucosal surface of the nasal cavity obtained using a nasal spray device and for analysis for detecting an analyte in the sample obtained as well as a test kit.

### Background Art

When samples are collected from human bodies for clinical testing, infectious disease testing, and other purposes, a wide variety of samples, such as samples of blood, saliva, feces, biopsy tissue, conjunctival swab specimens, nasopharyngeal swab and nasal swab specimens, pharyngeal swab specimens, nasal aspirates, nasal washing liquid, and nasal secretions collected by nose blowing, have heretofore been used.

When testing for infectious diseases represented by the influenza virus, for example, nasopharyngeal swab and nasal swab specimens, pharyngeal swab specimens, nasal aspirates, and nasal secretions collected by nose blowing are extensively used, and the presence or absence of the influenza virus antigen in a sample can be rapidly and simply determined using the test kit using the collected sample. During the influenza season, accordingly, samples are collected and used for testing on a regular basis (Patent Document 1).

In general, nasopharyngeal swab and nasal swab specimens are collected using sterilized medical swabs. While sample collection using swabs is regarded to be less invasive, it is necessary to insert a swab to the nasopharynx in order to obtain a sufficient amount of a sample for testing and to strongly scrape the mucosal membrane to obtain a sufficient amount of a sample. In some cases, accordingly, such sample collection may be accompanied by severe pain and bleeding. When collecting pharyngeal swab specimens, it is necessary to strongly scrape the palatine tonsil or the posterior pharyngeal wall to collect a sample, and a vomiting reflex may be induced in some cases. Thus, it may be difficult to collect a sufficient amount of a sample. Also, use of nasal aspirates is limited to some medical institutions equipped with special suction apparatuses. In addition, some patients have substantially no runny nose depending on their conditions, and it may be difficult to collect a sufficient amount of a sample. A large amount of a nasal washing liquid is introduced into the nasal cavity with the use of a syringe, and a discharged liquid is collected with the use of a paper cup or a suction apparatus as in the case of the nasal aspirates. When a large amount of a liquid is introduced into the nasal cavity, however, a patient may feel suffocated, and the introduced liquid may occasionally flow from the nose to the throat. Thus, such sample collection is not easy. Use of nasal secretions collected by nose blowing is selectively applicable to patients who are mature enough to blow their noses. As in the case of the nasal aspirates, accordingly, it may be difficult to collect a sufficient amount of a sample depending on patient's conditions. As described above, collection of 5 types of samples described above used for influenza virus detection; i.e., nasopharyngeal swab and nasal swab specimens, pharyngeal swab specimens, nasal aspirates, nasal washing liquid, and nasal secretions collected by nose blowing, is generally stressful for patients. In addition, a given amount of a sample cannot be obtained depending on patient's conditions, and test accuracy may be adversely affected. Accordingly, a method for sample collection and a test kit that enable stable collection of a given amount of a sample in a less-invasive manner have been awaited.

Also, collection of the 5 types of samples described above used for influenza virus detection increases the amount of biogenic substances to be included. This may induce unspecific responses, such as the false-positive, false-negative, or indeterminate outcome, depending on biological components. In the case of a nasal wash liquid, in particular, use of a large amount of a wash liquid would dilute the sample, and sensitivity would be deteriorated.

### Prior Art Documents

T. L. Noah and S. Becker, clinical immunology, 97, 1, 2000, 43 - 49 relates to a method to determine time courses of upregulation of several chemokines in nasal secretions after inoculation of human subjects with a low dose of live respiratory syncytial virus.
M. Velazquez-Gomez and S. Lacorte, environmental research, 178, 2019, 108726 relates to a method using nasal lavages as a non-invasive sample to determine in a direct way pollutant intake through inhalation.
Fujimoto et al., auris nasus larynx, 36, 3, 2009, 300 - 304 relates to a method, wherein the nasal cavity is misted over with saline, and the nasal lavage fluid is aspirated from the nostrils through a silicon tube.

### Patent Documents

Patent Document 1: WO 2005/121794
US 2005/0048465 A1 relates to a method of collecting nasopharyngeal specimens comprising the steps of: 1) positioning the patient with his or her face pointing upwards, 2) dropping at least a single drop of a sterile solution into each eye at a time and then holding head erect and blinking several times, 3) repeating step 2 until about 2-5 mL of solution has been used up, and 4) having the patient spit out the collected fluid in the back of the throat into a specimen container and relates to a kit to be used for the method.
US 2007/0184495 A1 relates to a nasal secretion assay kit comprising: a. a nasal secretion gathering means; and b. a reagent which contains one or more substances which will react with the nasal secretion to determine whether said nasal secretion is caused by an allergic reaction or a non-allergic reaction.
US 2018/0110499 A1 relates to a nasal irrigation diagnostic assembly comprising: an irrigation device including a fluid collection portion, said collection portion disposed and structured to retain a biological sample, in the form of waste solution from the nasal cavity resulting from irrigation, a detection member disposed on said irrigation device in exposed relation to the biological sample, within said collection portion, said detection member structured to determine the existence of at least one analyte biological sample of the waste solution, and whereby the at least one analyte comprises a pathogen/pathogen specific protein.
JP 2006/118936 relates to a flow-through membrane ELISA for detection of viral antigens in samples of nasal lavage.
JP 2008/539733 relates to a method for detection of Influenza virus in clinical samples based on nucleic acid amplification and detection.

### Summary of the Invention

### Objects to Be Attained by the Invention

The present invention provides a method for analyzing an analyte in a sample in a rapid and simple manner with high accuracy, which comprises collecting a mucosal fluid adhered to the mucosal surface of the nasal cavity in a less-invasive and simple manner with the use of a nasal spray device and using the collected mucosal fluid as a sample as well as a test kit.

### Means for Attaining the Objects

The present inventors have conducted concentrated studies and, as a consequence, discovered the following. That is, a sample of interest could be collected in a less invasive, simple, and stable manner by spraying a given amount of physiological saline to the nasal cavity with the use of a nasal spray device and collecting the adherent mucosal fluid obtained by washing the mucosal surface of the nasal cavity. They also found that the usefulness of such sample as a clinical sample by establishing an assay system that would detect a pathogen in the collected sample with sufficient sensitivity. On the basis of such finding, the present inventors have completed the present invention that would enable analysis of an analyte in a sample collected in a simple and less invasive manner.

The invention is defined in the appended claims.

### Effects of the Invention

According to the present invention, a method for diagnosis of an infectious disease with the use of a sample containing the adherent mucosal fluid obtained by washing the mucosal surface of the nasal cavity was established.

When an infectious disease is diagnosed with the use of, as a sample, the adherent mucosal fluid obtained by washing the mucosal surface of the nasal cavity according to the method of the present invention, an infectious disease can be diagnosed with sensitivity equivalent to or higher than the sensitivity of the diagnosis of an infectious disease with the use of, as a sample, nasopharyngeal swab and nasal swab specimens, pharyngeal swab specimens, nasal aspirates, nasal washing liquid, or nasal secretions collected by nose blowing.

### Embodiments of the Invention

Hereafter, the present invention is described in detail.

The present invention comprises spraying a given amount of a washing liquid such as physiological saline to the nasal cavity with the use of a nasal spray device and collecting, as a sample, the adherent mucosal fluid obtained by washing the mucosal surface of the nasal cavity. The term "adherent mucosal fluid" used herein refers to a mucosal fluid adhered to the mucosal surface of the nasal cavity.

For example, a given amount of a wash liquid is sprayed or applied dropwise to the nasal cavity of a subject, who is a patient suspected of an infectious disease on the basis of the inquiry, with the use of a nasal spray device filled with the washing liquid in advance. Thereafter, the mucosal surface of the nasal cavity is washed with the use of the washing liquid and the washing liquid containing the adherent mucosal fluid discharged to the entrance of the nose is collected as a sample. With the use of the sample thus obtained, various tests are performed.

According to the present invention, a washing liquid is sprayed to the nasal cavity to wash the mucosal surface of the nasal cavity, and it is thus possible to collect, as a sample, the washing liquid containing the adherent mucosal fluid discharged to the entrance of the nose.

When a washing liquid is introduced into the nasal cavity with the use of an instrument equipped with a dropper or a nozzle, the nasal cavity is topically washed. Thus, it is impossible to collect a mucosal fluid from an extensive area on the mucosal surface of the nasal cavity.

When a washing liquid is sprayed to the nasal cavity, in contrast, it is possible to collect a mucosal fluid from an extensive area on the mucosal surface of the nasal cavity.

When a washing liquid is introduced into the nasal cavity with the use of an instrument equipped with a dropper or a nozzle, the washing liquid passes through the nasal cavity to the throat due to the force of liquid flow, and it is impossible to collect the washing liquid efficiently.

When a washing liquid is sprayed to the nasal cavity, in contrast, it is possible to prevent the washing liquid from passing through the nasal cavity to the throat and to efficiently collect the washing liquid.

When a washing liquid is introduced into the nasal cavity with the use of an instrument equipped with a dropper or a nozzle, the washing liquid is introduced topically into the nasal cavity. Such force of liquid flow becomes a stress to the nasal cavity, and a patient (a child, in particular) often feels pain or suffocated.

When a washing liquid is sprayed to the nasal cavity, in contrast, the stress to the nasal cavity can be reduced, and a patient (a child, in particular) is less likely to feel pain or suffocated.

In the present invention, a nasal spray device is a container filled with a washing liquid such as physiological saline. Examples of nasal spray devices that can be used in the present invention include, but are not limited to, commercially available nasal spray containers, sprays, and medical spray devices.

An example of a washing liquid is physiological saline. Examples of physiological saline that can be used include, but are not limited to, commercially available physiological saline specified by the Japanese Pharmacopoeia and physiological saline prepared in accordance with the prescription of the Japanese Pharmacopoeia. Other examples of a washing liquid that can be used include isotonic fluids, such as a Ringer's solution, a lactated Ringer's solution, and dextrose in water.

The nasal cavity is washed with the use of a washing liquid in an amount of 300 to 500 µl by spraying the nasal cavity with a single-use nasal spray device.

A fluid containing the adherent mucosal fluid discharged to the entrance of the nose can be collected as a sample with the use of various collection instruments, such as instruments that can support fluid, in the form of absorbers (e.g., cotton swabs, swabs, brushes, instrument with mesh-like edges, spongy instruments, and cloths). A fluid containing the adherent mucosal fluid may be absorbed by such instrument and collected.

In the present invention, a sample thus collected is referred to as an "easily-collectible sample of a mucosal fluid adhered to the mucosal surface of the nasal cavity."

The sample collected with the use of a collection instrument may be dissolved or suspended in a fluid, and the resulting fluid may be used as a sample fluid. The sample collected with the use of a collection instrument can be dissolved or suspended in a fluid by, for example, squeezing, stroking, or scrubbing a portion of the collection instrument that have absorbed a fluid containing the adherent mucosal fluid, such as the cotton portion at the end of a cotton swab. As a fluid in which the sample is to be dissolved or suspended, a buffer can be used.

In the present invention, a sample can be used without any treatment, such as concentration or culture. Alternatively, a sample may be mixed with a buffer and used in that state. An example of a buffer that can be used is phosphate buffer, and a buffer may contain a surfactant, such as Tween 20, or serum albumin. When an immunochromatographic assay, which is a lateral-flow immunoassay using a membrane, is to be performed, for example, a sample collected with the use of a cotton swab is suspended in a buffer and used as a sample solution.

Disclosed infectious diseases to be detected are infectious diseases caused by pathogens, such as viruses, bacteria, protozoans, fungi, Mycoplasma, Rickettsia, and chlamydia. In particular, infectious diseases caused by viruses are to be detected. Infectious diseases to be detected according to the invention are caused by influenza viruses, coronaviruses, such as SARS-CoV, MERS-CoV, and SARS-CoV-2, RS viruses, adenoviruses, and human metapneumoviruses. Pathogens causing such infectious diseases may be detected as analytes.

Examples of methods for detecting analytes include immunoassays (immunological assays), genetic screening, and separation culture/identification.

In immunoassays, viruses in a sample are analyzed based on the antibody-antigen reaction. Specifically, viruses can be analyzed with the use of antibodies specific to virus antigens. Antibodies specific to virus antigens can be obtained in accordance with a conventional technique. As a technique of immunoassays, a person skilled in the art can adopt any well-known method in the art, such as immunostaining, including the fluorescence antibody technique, the enzyme antibody technique, the heavy metal-labeled antibody technique, and the radioisotope-labeled antibody technique, techniques involving separation by electrophoresis in combination with detection using fluorescence, enzyme, and radioisotope (e.g., Western blotting and two-dimensional fluorescence electrophoresis), enzyme-linked immunosorbent assay (ELISA), dot-blotting, latex agglutination-turbidimetric immunoassays (LA), and immunochromatography. In the present invention, the term "analysis" include quantification, semi-quantification, and detection.

Among the immunoassay techniques described above, the sandwich technique is particularly preferable. The sandwich technique is well known in the field of immunoassays and it can be carried out by, for example, lateral-flow immunochromatographic assays or ELISA. Such sandwich techniques are well known in the art, and the method of the present invention can be carried out in accordance with the well-known sandwich technique.

In immunoassay techniques based on the sandwich technique, any solid-phase supports on which antibodies can be immobilized in accordance with a conventional technique can be used. For example, any known supports, such as porous membranes having capillary actions, particulate substances, test tubes, and resin plates, can be selected. Examples of substances that label antibodies include enzymes, radioisotopes, fluorescent substances, luminescent substances, colored particles, and colloid particles. Two or more types of antibodies may be used. Two or more types of antibodies are preferably used in the sandwich technique, and an epitope recognized by an antibody is preferably different from an epitope recognized by another antibody.

Among the immunoassay techniques performed with the use of various materials described above, the lateral-flow immunochromatography assay technique using a membrane is particularly preferable from the viewpoint of simplicity and rapidity in clinical testing.

The lateral-flow immunoassay technique according to the method of the present invention is performed with the use of an immunoassay apparatus comprising: a support having a detection region on which an antibody (Antibody 1) that captures a target (an antigen) is immobilized; a label region having a mobile label antibody (Antibody 2) labeled with an adequate label such as a colored polystyrene particle or gold colloid; a sample pad onto which a sample is added dropwise; an absorption band that absorbs a developed sample solution; and a backing sheet that assembles such members to form a laminated body. In such method, a washing liquid is applied to the nasal cavity of a subject suspected of an infectious disease to wash the mucosal surface of the nasal cavity, a sample, which is the washing liquid containing a mucosal fluid adhered to the mucosal surface of the nasal cavity, is added dropwise to a sample pad, and a composite of Antibody 2 capable of binding to the target (a label reagent) labeled with an adequate label, such as a colored polystyrene particle or gold colloid, and the analyte is allowed to stretch and migrate to a solid-phase support comprising Antibody 1 immobilized thereon with the use of capillary actions. As a result, the complex of the immobilized substance-analyte-label reagent is formed on the solid-phase support, and the analyte can be detected by detecting a signal of the label reagent emitted from the complex (the solid-phase support comprising the substance capable of binding to the analyte immobilized thereon becomes red in the case of gold colloid). The immunoassay technique can be performed at 5°C to 35°C, and preferably at room temperature, and a pretreatment with the use of a sample treatment solution may also be performed within such temperature range.

The number of detection regions and types of label antibodies to be contained in the label region are not limited to 1. With the use of an antibody reacting with a plurality of analytes, 2 or more antigens can be detected with the use of the same immunoassay apparatus.

The present invention also relates to an immunoassay reagent, which is the immunoassay instrument for analyzing an analyte in a sample and detecting an infectious disease and an immunoassay test kit comprising the immunoassay reagent and a nasal spray device.

When the number of analytes, such as viruses, is small, the number of analytes may be first increased via culture by the method of separation culture/identification and may then be analyzed.

Examples of gene screening techniques include gene amplification techniques, such as PCR (polymerase chain reaction), LAMP (loop mediated isothermal amplification), TMA (transcription mediated amplification), SDA (strand displacement amplification), and ICAN (isothermal and chimeric primer-initiated amplification of nucleic acids). Among such techniques, PCR is particularly preferable. A washing liquid may be applied to the nasal cavity of a subject suspected of an infectious disease to wash the mucosal surface of the nasal cavity, the washing liquid containing a mucosal fluid adhered to the mucosal surface of the nasal cavity is designated as a sample, and the gene of the pathogen contained in the sample may be amplified. In PCR, a gene region surrounded by primers having particular sequences is amplified *in vitro* with the use of the Taq DNA polymerase reaction. A gene amplification reaction is performed with the use of a pair of primers; i.e., a forward primer and a reverse primer, by repeating a 3-step thermal cycle: i) thermal denaturation of double-stranded DNA; ii) annealing of primers; and iii) elongation, 30 to 40 times. Techniques described above are known techniques.

### Examples

Hereafter, the present invention is described in greater detail with reference to the examples, although the present invention is not limited to the examples.

### Example 1

### 1. Preparation of nasal spray device

A 20-ml nasal spray container (Kinshi Seisakusho) was filled with 10 ml of physiological saline (Otsuka Pharmaceutical Co., Ltd.) to prepare a nasal spray device.

### 2. Collection of easily-collectible samples of mucosal fluids adhered to the mucosal surface of the nasal cavity

Washing liquids in amounts of 200 µl, 300 µl, 400 µl, 500 µl, 600 µl, 700 µl, and 800 µl were sprayed to the nasal cavity of each of 3 healthy adult subjects with the use of the nasal spray device, the liquids discharged to the entrance of the nose were absorbed to Ex Swabs 003T and collected, and the collected liquids were designated as easily-collectible samples of mucosal fluids adhered to the mucosal surface of the nasal cavity.

### 3. Functional test at the time of collection

When collecting the easily-collectible samples of mucosal fluids adhered to the mucosal surface of the nasal cavity in 2. above, the 3 healthy adult subjects were subjected to evaluation in terms of ease of collection, a feeling of suffocation, and difficulty in fluid flow to the throat at each spray dose.

### 4. Comparison and Examination

On the basis of the results shown in Table 1, the optimal spray dose was considered to be 300 to 500 µl, although the optimal spray dose would vary among individuals. Because healthy adult subjects were employed, different results may be obtained in the case of child subjects. In addition, an optimal dose may vary among races. Thus, the optimal dose is not limited to the dose indicated above.

**Table 1**

| Spray dose (µl) | Evaluation points in functional test | 3 Healthy subjects | | |
|---|---|---|---|---|
| | | A | B | C |
| 200 | Ease of collection | Fine | Poor | Fine |
| | Feeling of suffocation | Very good | Very good | Very good |
| | Difficulty in fluid flow to throat | Very good | Very good | Very good |
| 300 | Ease of collection | Very good | Good | Good |
| | Feeling of suffocation | Very good | Good | Very good |
| | Difficulty in fluid flow to throat | Very good | Very good | Very good |
| 400 | Ease of collection | Very good | Very good | Very good |
| | Feeling of suffocation | Very good | Fine | Very good |
| | Difficulty in fluid flow to throat | Very good | Fine | Very good |
| 500 | Ease of collection | Very good | Very good | Very good |
| | Feeling of suffocation | Very good | Poor | Very good |
| | Difficulty in fluid flow to throat | Good | Poor | Good |
| 600 | Ease of collection | Very good | Very good | Good |
| | Feeling of suffocation | Good | Poor | Very good |
| | Difficulty in fluid flow to throat | Fine | Poor | Fine |
| 700 | Ease of collection | Good | Very good | Fine |
| | Feeling of suffocation | Good | Poor | Very good |
| | Difficulty in fluid flow to throat | Fine | Poor | Poor |
| 800 | Ease of collection | Fine | Very good | Fine |
| | Feeling of suffocation | Good | Poor | Very good |
| | Difficulty in fluid flow to throat | Fine | Poor | Poor |

### Example 2

### 1. SDS-PAGE analysis of easily-collectible samples of mucosal fluids adhered to the mucosal surface of the nasal cavity

The easily-collectible samples of mucosal fluids adhered to the mucosal surface of the nasal cavity collected in Example 1 2. were suspended in 200 µl of physiological saline. The samples were fractionated, reagents were added thereto to adjust the final concentration to 62.5 mM Tris-HCl (pH 6.5), 10(w/v)% glycerol, 2.3(w/v)% SDS, and 0.05(w/v)% BPB (dye), the resultant was subjected to thermal denaturation at 95°C for 5 minutes, and SDS-PAGE was performed in accordance with a conventional technique.

### 2. Analysis of total protein level using densitometry analyzer

With the use of a densitometry analyzer (BioRad), the CBB-stained gel obtained in SDS-PAGE performed in 1. above was analyzed, the band density in each lane was assayed, and the total density level was designated as the density score.

### 3. Comparison and Examination

Table 2 shows the results of measurement of the density score. While the results of evaluation varied among individuals, as shown in Table 2, the protein levels in the easily-collectible samples of mucosal fluids adhered to the mucosal surface of the nasal cavity at a spray dose of 400 to 700 µl were found to be high. Because healthy adult subjects were employed, different results may be obtained in the case of patients with symptoms or child subjects. In addition, an optimal dose may vary among races. Thus, the optimal dose is not limited to the dose indicated above.

**Table 2**

| Spray dose (µl) | Density Score | | |
|---|---|---|---|
| | 3 Healthy subjects | | |
| | A | B | C |
| 200 | 106.4 | 104.6 | 107.5 |
| 300 | 110.5 | 105.9 | 119.0 |
| 400 | 109.5 | 110.1 | 152.4 |
| 500 | 119.2 | 106.1 | 113.4 |
| 600 | 129.5 | 102.0 | 100.0 |
| 700 | 100.0 | 103.8 | 127.8 |
| 800 | 106.8 | 100.0 | 101.1 |

### Example 3

### 1. SDS-PAGE analysis of nasal aspirate samples and easily-collectible samples of mucosal fluids adhered to the mucosal surface of the nasal cavity

Samples were collected from 10 nasal aspirate samples using Mentip P1503 (J.C.B. Industry Limited) and suspended in 200 µl of physiological saline to prepare SDS-PAGE samples. Also, the easily-collectible samples of mucosal fluids adhered to the mucosal surface of the nasal cavity collected from 3 healthy subjects by spraying 400 µl of the liquidss in 2. of Example 1 were mixed to prepare SDS-PAGE samples. The samples were fractionated, reagents were added thereto to adjust the final concentration to 62.5 mM Tris-HCl (pH 6.5), 10(w/v)% glycerol, 2.3(w/v)% SDS, and 0.05(w/v)% BPB (dye), the resultants were subjected to thermal denaturation at 95°C for 5 minutes, and SDS-PAGE was performed in accordance with a conventional technique.

### 2. Analysis of total protein level using densitometry analyzer

With the use of a densitometry analyzer (BioRad), the CBB-stained gel obtained in SDS-PAGE performed in 1. above was analyzed, the band density in each lane was assayed, and the total density level (the density score) was determined.

### 3. Comparison and Examination

Table 3 shows the results of measurement of the density scores. While the results varied among samples, as shown in Table 3, no significant differences were observed in the amount of the collected proteins between the samples of the nasal aspirates and the easily-collectible samples of mucosal fluids adhered to the mucosal surface of the nasal cavity when the samples were mixed. It was thus considered that influenza viruses could be tested with the use of the easily-collectible samples of mucosal fluids adhered to the mucosal surface of the nasal cavity according to the present invention.

**Table 3**

| Samples | Density Score |
|---|---|
| Easily-collectible sample of mucosal fluid adhered to mucosal surface of nasal cavity | 153.5 |
| Nasal aspirate 1 | 144.0 |
| Nasal aspirate 2 | 115.9 |
| Nasal aspirate 3 | 201.0 |
| Nasal aspirate 4 | 140.8 |
| Nasal aspirate 5 | 96.5 |
| Nasal aspirate 6 | 118.0 |
| Nasal aspirate 7 | 120.2 |
| Nasal aspirate 8 | 102.0 |
| Nasal aspirate 9 | 187.5 |
| Nasal aspirate 10 | 176.2 |
| Mixture of nasal aspirates 1 to 10 | 161.0 |

### Example 4

### 1. Preparation of anti-influenza A virus antibodies

BALB/c mice were immunized with inactivated influenza A viruses and raised for a given period of time. The spleens were removed from the mice and fused to mouse myeloma cells (P3X63) in accordance with the method of Kohler et al. (Kohler et al., Nature, vol. 256, pp. 495-497, 1975). The resulting fused cells (hybridomas) were maintained in an incubator at 37°C, and cells were purified (monoclonalized) while observing the antibody activity of the supernatant by ELISA using a plate comprising an antigen extracted from *Helicobacter pylori* immobilized thereon. The 2 resulting cell lines were administered intraperitoneally to the pristane-treated BALB/c mice, and the antibody-containing ascites fluids were collected approximately 2 weeks later.

IgG was purified from the obtained ascites by affinity chromatography using a protein A column to obtain 2 types of purified anti-influenza A virus antibodies.

### 2. Preparation of anti-influenza B virus antibodies

BALB/c mice were immunized with inactivated influenza B viruses and raised for a given period of time. The spleens were removed from the mice and fused to mouse myeloma cells (P3X63) in accordance with the method of Kohler et al. (Kohler et al., Nature, vol. 256, pp. 495-497, 1975). The resulting fused cells (hybridomas) were maintained in an incubator at 37°C, and cells were purified (monoclonalized) while observing the antibody activity of the supernatant by ELISA using a plate comprising an antigen extracted from *Helicobacter pylori* immobilized thereon. The 2 resulting cell lines were administered intraperitoneally to the pristane-treated BALB/c mice, and the antibody-containing ascites fluids were collected approximately 2 weeks later.

IgG was purified from the obtained ascites by affinity chromatography using a protein A column to obtain 2 types of purified anti-influenza B virus antibodies.

### 3. Preparation of labeled anti-influenza A virus antibodies

One of the anti-influenza A virus antibodies was dialyzed against a 50 mM MES (2-morpholinoethanesulfonic acid, monohydrate, Dojindo Laboratories) buffer (pH 6.0) solution, and 10 ml of a solution was prepared by diluting the dialyzed antibody solution with the same buffer to adjust O.D. 280 nm to 0.5. Subsequently, the resultant was mixed with 10(w/v)% blue polystyrene latex particles (particle diameter: 0.45 µm; surface functional group: carboxyl; functional group density: 65Å2/COOH group; Magsphere) to result in the liquid volume ratio of 40:1, and the reaction was allowed to proceed. Subsequently, 1(w/v)% EDAC (N-(3-dimethlaminopropyl)-N'-ethylcarbodiimide hydrochloride (Sigma)) was added to the final concentration of 0.1%, and the reaction was then allowed to proceed for 2 hours. The reaction product was washed, suspended in 20 ml of the final suspension (5 mM Tris, 0.04(w/v)% BSA (bovine serum albumin), 0.4 M trehalose, 0.2(v/v)% TritonX-100), and applied to an ultrasonic dispersion apparatus (Olympus) to disperse latex particles.

### 4. Preparation of labeled anti-influenza B virus antibodies

One of the anti-influenza B virus antibodies was dialyzed against a 50 mM MES (2-morpholinoethanesulfonic acid, monohydrate, Dojindo Laboratories) buffer (pH 6.0) solution, and 10 ml of a solution was prepared by diluting the dialyzed antibody solution with the same buffer to adjust O.D. 280 nm to 0.5. Subsequently, the resultant was mixed with 10(w/v)% blue polystyrene latex particles (particle diameter: 0.45 µm; surface functional group: carboxyl; functional group density: 65Å2/COOH group; Magsphere) to result in the liquid volume ratio of 40:1, and the reaction was allowed to proceed. Subsequently, 1(w/v)% EDAC (N-(3-dimethlaminopropyl)-N'-ethylcarbodiimide hydrochloride (Sigma)) was added to the final concentration of 0.1%, and the reaction was then allowed to proceed for 2 hours. The reaction product was washed, suspended in 20 ml of the final suspension (5 mM Tris, 0.04(w/v)% BSA (bovine serum albumin), 0.4 M trehalose, 0.2(v/v)% TritonX-100), and applied to an ultrasonic dispersion apparatus (Olympus) to disperse latex particles.

### 5. Preparation of latex particle-labeled antibody drying pad

The latex particle-labeled anti-influenza A and B virus antibodies obtained in 3. and 4. above were mixed and sprayed on the entire surface of a cellulose nonwoven fabric having a width of 15 mm wound in a reel shape at a coating amount of 8 µl/cm using a positive pressure spray device (BioJet; BioDot). Thereafter, warm air at 50°C was blown for 1 minute to dry, thereby preparing a latex particle-labeled antibody drying pad.

### 6. Preparation of antibody for solid-phase membrane

The purified anti-influenza A virus antibody prepared in 1. above, which was not used for labeling, was dialyzed against a solid solution (10 mM Tris-HCl (pH 8.0)), followed by filtration with a 0.22 µm filter. The solution was diluted with a solid solution to adjust OD 280 nm to 3.0, and an anti-influenza A virus antibody for solid phase was prepared.

The purified anti-influenza B virus antibody prepared in 2. above, which was not used for labeling, was dialyzed against a solid solution (10 mM Tris-HCl (pH 8.0)), followed by filtration with a 0.22 µm filter. The solution was diluted with a solid solution to adjust OD 280 nm to 3.0, and an anti-influenza B virus antibody for solid phase was prepared.

### 7. Preparation of lateral-flow membrane assay device for detection of influenza viruses

A nitrocellulose membrane (pore diameter: 12 µm; Whatman) sheet (white) having a width of 3 cm and a length of 10 cm was used. With the use of a positive pressure spray apparatus (BioJet; BioDot), the anti-influenza A virus antibody for solid phase was applied linearly at a position 6 mm away from one end of the long axis (this end is the upstream end and the opposite side is the downstream end) at a coating amount of 1 µl/cm, the anti-influenza B virus antibody for solid phase was applied linearly at a position 8 mm away from one end of the long axis at a coating amount of 1 µl/cm, and the anti-mouse IgG antibody diluted to OD 280 nm of 1.0 was applied linearly at a position 13 mm away from one end of the long axis at a coating amount of 1 µl/cm. Thereafter, warm air at 45°C was brown for 30 minutes to dry the membrane.

In order to fix the member and increase the strength, subsequently, a plastic backing sheet (BioDot) was bonded to the opposite side (this surface is the lower surface) of the membrane surface on which the antibody has been applied (this surface is the upper surface).

Subsequently, the latex particle-labeled antibody drying pad prepared in 5. above was cut into a width of 15 mm and a length of 10 cm, so that the upstream end of the membrane would overlap with the upper surface of the membrane by 2 mm, and a cellulose filter paper (Wattman) having a width of 23 mm and a length of 10 cm was applied on top of the latex particle-labeled antibody drying pad to overlap by 13 mm. Thus, a sample application pad was prepared.

Subsequently, a cellulose filter paper (Wattman) having a width of 30 mm and a length of 10 cm was applied on top of the membrane to overlap with the downstream end of the membrane by 5 mm. Thus, a sample absorption pad was prepared.

Subsequently, the entire upper surface was covered with a transparent plastic laminate (Adhesive Research) except for a width of 5 mm at the upstream end of the sample application pad.

In the end, the resultant was cut along the long axis by 5 mm to prepare a membrane assay apparatus.

### 8. Detection of influenza viruses

Nasopharyngeal swab and nasal swab specimens were collected using an influenza virus antigen test kit, QuickNave^{™}-Flu 2 (Denka Company Limited), for diagnosis of influenza virus infection. Five patients diagnosed positive(+) for the influenza A virus, five patients diagnosed positive(+) for the influenza B virus, and five patients diagnosed negative(-) for the influenza viruses on the basis of the overall finding were designated as the subjects. To the nasal cavity of each subject, 400 µl of physiological saline was sprayed with the use of the nasal spray device prepared in 1 of Example 1, the liquids discharged to the entrance of the nose were absorbed to Ex Swabs 003T (Denka Company Limited) and collected, and the collected liquids were designated as easily-collectible samples of mucosal fluids adhered to the mucosal surface of the nasal cavity. The tips of the cotton swabs used for collecting the samples were soaked in 0.2 ml of a sample suspension buffer (a phosphate buffer (pH 7.4) containing Tween 20 (0.05(w/v)%) and bovine serum albumin (0.1(w/v)%)), and substances adhered to the tips were squeezed out into the sample suspension buffer to prepare a sample solution.

The sample application pad of the lateral-flow membrane assay apparatus for influenza virus detection prepared in 7. was soaked in the sample solution. The assay apparatus was inspected 10 minutes later. The case in which color development was observed at the position (control line) where the anti-mouse IgG antibody had been applied was regarded as valid. When color development was observed at the position where the anti-influenza A virus antibody for solid-phase had been applied, the subject was evaluated positive(+) for the influenza A virus. When color development was observed at the position where the anti-influenza B virus antibody for solid-phase had been applied, the subject was evaluated positive(+) for the influenza B virus. When color development was not observed in any position, the subject was evaluated negative(-). The case in which no color development was observed on the control line was regarded as invalid.

### 9. Comparison and Examination

Table 4 shows the results of assays. The results shown in Table 4 demonstrate that all the test results attained with the samples according to the present invention were consistent on the basis of comparison of the results of the test performed by collecting nasopharyngeal swab and nasal swab specimens using QuickNave^{™}-Flu 2 and the overall findings.

**Table 4**

| Sample No. | Lateral-flow membrane assay apparatus | | Overall finding |
|---|---|---|---|
| | QuickNavi-Flu2 nasopharyngeal swab and nasal swab specimen | Easily-collectible sample of mucosal fluid adhered to mucosal surface of nasal cavity | |
| No. 1 | - | - | - |
| No. 2 | A+ | A+ | A+ |
| No. 3 | - | - | - |
| No. 4 | B+ | B+ | B+ |
| No. 5 | A+ | A+ | A+ |
| No. 6 | B+ | B+ | B+ |
| No. 7 | A+ | A+ | A+ |
| No. 8 | A+ | A+ | A+ |
| No. 9 | - | - | - |
| No. 10 | B+ | B+ | B+ |
| No. 11 | - | - | - |
| No. 12 | B+ | B+ | B+ |
| No. 13 | A+ | A+ | A+ |
| No. 14 | B+ | B+ | B+ |
| No. 15 | - | - | - |

### Example 5 (comparative example)

### 1. Sample collection for comparison of influenza virus levels

Nasopharyngeal swab and nasal swab specimens were collected using an influenza virus antigen test kit, QuickNave^{™}-Flu 2 (Denka Company Limited), for diagnosis of influenza virus infection. To the nasal cavity of a subject diagnosed positive(+) for the influenza A virus on the basis of the overall finding, 400 µl of physiological saline was sprayed with the use of the nasal spray device prepared in 1. of Example 1., the liquid discharged to the entrance of the nose was absorbed to Ex Swabs 003T (Denka Company Limited) and collected as an easily-collectible sample of a mucosal fluid adhered to the mucosal surface of the nasal cavity, and the collected sample was suspended in 200 µl of physiological saline to prepare a qPCR sample. The residual liquid of the nasopharyngeal swab and nasal swab specimens obtained from the same subject with the use of QuickNave^{™}-Flu 2 was used as a control sample.

### 2. Real-time PCR analysis

With the use of the QIAamp Viral RNA Mini Kit (a kit for nucleic acid extraction, QIAGEN), nucleic acids were extracted from the 2 samples obtained in 1. above and analyzed using the PCR apparatus (Applied Biosystems QuantStudio^{™} 3, Thermo Fisher Scientific) with the addition of a given PCR sample.

### 3. Comparison and Examination

Table 5 shows the results of assays. The results shown in Table 5 demonstrate that the virus level in the easily-collectible sample of a mucosal fluid adhered to the mucosal surface of the nasal cavity was equivalent to 1.39 × 10⁶ copies/ml and the virus level in the control sample of nasopharyngeal swab and nasal swab specimens was equivalent to 1.31 × 10⁵ copies/ml. The virus level in the easily-collectible sample of a mucosal fluid adhered to the mucosal surface of the nasal cavity was higher than the virus level in the nasopharyngeal swab and nasal swab specimens according to a conventional technique. This indicates that the easily-collectible sample of a mucosal fluid adhered to the mucosal surface of the nasal cavity enables more stable virus collection compared with the nasopharyngeal swab and nasal swab specimens and that such easily-collectible sample can be used for clinical testing.

**Table 5**

| Sample No. | Real time PCR analysis | |
|---|---|---|
| | QuickNavi-Flu2 nasopharyngeal swab and nasal swab specimen | Easily-collectible sample of mucosal fluid adhered to mucosal surface of nasal cavity |
| No. 16 | 1.31 × 10⁵ | 1.39 × 10⁶ |

### Industrial Applicability

The present invention can be used for detection of respiratory infectious diseases.

## Claims

1. A method for detecting an infectious disease caused by influenza virus, coronavirus, RS virus, adenovirus, or human metapneumovirus by a lateral-flow immunoassay with the use of an immunoassay apparatus comprising members that are a solid-phase support having a detection region on which an antibody specific to an antigen of influenza virus, coronavirus, RS virus, adenovirus, or human metapneumovirus is immobilized, a label region having a mobile label antibody specific to an antigen of influenza virus, coronavirus, RS virus, adenovirus, or human metapneumovirus, a sample pad and an absorption band, and further comprising a backing sheet that assembles such members to form a laminated body, the method comprising
spraying a washing liquid to the nasal cavity of a subject suspected of the infectious disease to wash the mucosal surface of the nasal cavity using a nasal spray device is a single-use nasal spray device that contains a single dose of 300 to 500 µl of a wash liquid,
collecting, as a sample, the washing liquid containing the mucosal fluid adhered to the mucosal surface of the nasal cavity, wherein the amount of the washing liquid sprayed to the nasal cavity is 300 to 500 µl,
adding the sample to the sample pad, and
detecting the antigen of influenza virus, coronavirus, RS virus, adenovirus, or human metapneumovirus by detecting a signal of the label reagent emitted from the complex of the immobilized antibody-influenza virus antigen, coronavirus antigen, RS virus antigen, adenovirus antigen, or human metapneumovirus antigen-label reagent formed on the solid-phase support.

2. A test kit used for detecting an infectious disease caused by influenza virus, coronavirus, RS virus, adenovirus, or human metapneumovirus by spraying a washing liquid to the nasal cavity of a subject suspected of an infectious disease to wash the mucosal surface of the nasal cavity and using, as a sample, the collected washing liquid containing the mucosal fluid adhered to the mucosal surface of the nasal cavity,
which comprises a test reagent for detecting an infectious disease by a lateral-flow immunoassay which is an immunoassay apparatus comprising members that are a solid-phase support having a detection region on which an antibody specific to an antigen of influenza virus, coronavirus, RS virus, adenovirus, or human metapneumovirus is immobilized, a label region having a mobile label antibody specific to an antigen of influenza virus, coronavirus, RS virus, adenovirus, or human metapneumovirus, a sample pad and an absorption band, and further comprising a backing sheet that assembles such members to form a laminated body, and
a nasal spray device containing a washing liquid for washing the mucosal membrane of the nasal cavity, wherein the nasal spray device is a single-use nasal spray device and the device contains a single dose of 300 to 500 µl of a wash liquid to be sprayed to the nasal cavity.

## Patentansprüche

1. Verfahren zum Nachweis einer durch Influenzavirus, Coronavirus, RS-Virus, Adenovirus oder humanes Metapneumovirus verursachten Infektionskrankheit durch einen Lateral-Flow-Immunoassay unter Verwendung einer Immunoassay-Vorrichtung, umfassend Bestandteile, die ein Festphasenträger aufweisend einen Nachweisbereich, auf dem ein für ein Antigen von Influenzavirus, Coronavirus, RS-Virus, Adenovirus oder humanem Metapneumovirus spezifischer Antikörper immobilisiert ist, ein Markierungsbereich aufweisend einen mobilen, für ein Antigen von Influenzavirus, Coronavirus, RS-Virus, Adenovirus oder humanem Metapneumovirus spezifischen Markierungsantikörper, ein Probenpad und ein Absorptionsband sind, und ferner umfassend ein Trägerblatt, das solche Bestandteile zusammensetzt, um einen laminierten Körper zu bilden, wobei das Verfahren umfasst:
Sprühen einer Waschflüssigkeit in die Nasenhöhle eines Subjekts mit Verdacht auf die Infektionskrankheit, um die Schleimhautoberfläche der Nasenhöhle unter Verwendung einer Nasensprayvorrichtung, welche eine Einweg-Nasensprayvorrichtung ist, welche eine Einzeldosis von 300 bis 500 µl einer Waschflüssigkeit enthält, zu waschen,
Sammeln der Waschflüssigkeit, enthaltend die an der Schleimhautoberfläche der Nasenhöhle anhaftende Schleimhautflüssigkeit, als Probe,
wobei die in die Nasenhöhle gesprühte Menge der Waschflüssigkeit 300 bis 500 µl beträgt,
Zugeben der Probe zu dem Probenpad, und
Nachweisen des Antigens von Influenzavirus, Coronavirus, RS-Virus, Adenovirus oder humanem Metapneumovirus durch Nachweisen eines Signals des Markierungsreagenzes, das von dem auf dem Festphasenträger gebildeten Komplex aus immobilisiertem Antikörper-Influenzavirus-Antigen, Coronavirus-Antigen, RS-Virus-Antigen, Adenovirus-Antigen oder humanem Metapneumovirus-Antigen-Markierungsreagenz emittiert wird.

2. Testkit zur Verwendung zum Nachweis einer durch Influenzavirus, Coronavirus, RS-Virus, Adenovirus oder humanes Metapneumovirus verursachten Infektionskrankheit durch Sprühen einer Waschflüssigkeit in die Nasenhöhle eines Subjekts mit Verdacht auf eine Infektionskrankheit, um die Schleimhautoberfläche der Nasenhöhle zu waschen und Verwenden der gesammelten Waschflüssigkeit, enthaltend die an der Schleimhautoberfläche der Nasenhöhle anhaftende Schleimhautflüssigkeit, als Probe,
welches ein Testreagenz zum Nachweis einer Infektionskrankheit durch einen Lateral-Flow-Immunoassay umfasst, welcher eine Immunoassay-Vorrichtung ist, umfassend Bestandteile, die ein Festphasenträger aufweisend eine Nachweisbereich, auf dem ein für ein Antigen von Influenzavirus, Coronavirus, RS-Virus, Adenovirus oder humanem Metapneumovirus spezifischer Antikörper immobilisiert ist, einen Markierungsbereich aufweisend einen mobilen, für ein Antigen von Influenzavirus, Coronavirus, RS-Virus, Adenovirus oder humanem Metapneumovirus spezifischen Markierungsantikörper, ein Probenpad und ein Absorptionsband sind, und ferner umfassend ein Trägerblatt, das solche Bestandteile zusammensetzt, um einen laminierten Körper zu bilden,
und eine Nasensprayvorrichtung, enthaltend eine Waschflüssigkeit zum Waschen der Schleimhaut der Nasenhöhle,
wobei die Nasensprayvorrichtung eine Einweg-Nasensprayvorrichtung ist und die Vorrichtung eine Einzeldosis von 300 bis 500 µl einer in die Nasenhöhle zu sprühenden Waschflüssigkeit enthält.

## Revendications

1. Un procédé pour détecter une maladie infectieuse causée par un virus de la grippe, un coronavirus, un virus RS, un adénovirus ou un métapneumovirus humain par un essai immunologique à flux latéral en utilisant d'un appareil d'essai immunologique comprenant des éléments qui sont un support en phase solide ayant une région de détection sur laquelle est immobilisé un anticorps spécifique d'un antigène de virus de la grippe, de coronavirus, de virus RS, d'adénovirus ou de métapneumovirus humain, une région de marquage ayant un anticorps de marquage mobile spécifique d'un antigène de virus de la grippe, de coronavirus, de virus RS, d'adénovirus ou de métapneumovirus humain, un tampon d'échantillon et une bande d'absorption, et comprenant en outre une feuille de support qui assemble de tels éléments pour former un corps stratifié, le procédé comprenant :
vaporiser un liquide de lavage dans la cavité nasale d'un sujet suspecté de la maladie infectieuse pour laver la surface muqueuse de la cavité nasale à l'aide d'un dispositif de vaporisation nasale, le dispositif de vaporisation nasale étant un dispositif de vaporisation nasale à usage unique qui contient une dose unitaire de 300 à 500 µl d'un liquide de lavage,
recueillir, en tant qu'échantillon, le liquide de lavage contenant le fluide muqueux adhérant à la surface muqueuse de la cavité nasale,
dans lequel la quantité du liquide de lavage vaporisée dans la cavité nasale est de 300 à 500 µl,
ajouter l'échantillon au tampon d'échantillon, et
détecter l'antigène de virus de la grippe, de coronavirus, de virus RS, d'adénovirus ou de métapneumovirus humain en détectant un signal du réactif de marquage émis par le complexe de anticorps immobilisé-antigène de virus de la grippe, antigène de coronavirus, antigène de virus RS, antigène d'adénovirus ou antigène de métapneumovirus humain-réactif de marquage formé sur le support en phase solide.

2. Un kit de test utilisé pour détecter une maladie infectieuse causée par un virus de la grippe, un coronavirus, un virus RS, un adénovirus ou un métapneumovirus humain en vaporisant un liquide de lavage dans la cavité nasale d'un sujet suspecté d'une maladie infectieuse pour laver la surface muqueuse de la cavité nasale et en utilisant, en tant qu'échantillon, le liquide de lavage recueilli contenant le fluide muqueux adhérant à la surface muqueuse de la cavité nasale,
lequel comprend un réactif de test pour détecter une maladie infectieuse par un essai immunologique à flux latéral qui est un appareil d'essai immunologique comprenant des éléments qui sont un support en phase solide ayant une région de détection sur laquelle est immobilisé un anticorps spécifique d'un antigène de virus de la grippe, de coronavirus, de virus RS, d'adénovirus ou de métapneumovirus humain, une région de marquage ayant un anticorps de marquage mobile spécifique d'un antigène de virus de la grippe, de coronavirus, de virus RS, d'adénovirus ou de métapneumovirus humain, un tampon d'échantillon et une bande d'absorption, et comprenant en outre une feuille de support qui assemble de tels éléments pour former un corps stratifié,
et un dispositif de vaporisation nasale contenant un liquide de lavage pour laver la membrane muqueuse de la cavité nasale,
dans lequel le dispositif de vaporisation nasale est un dispositif de vaporisation nasale à usage unique et le dispositif contient une dose unitaire de 300 à 500 µl d'un liquide de lavage à vaporiser dans la cavité nasale.
